Europäisches Patentamt

European Patent Office

Office européen des brevets

(19)

(11) EP 1 299 089 B1

## (12) EUROPEAN PATENT SPECIFICATION

(45) Date of publication and mention
of the grant of the patent:
**12.04.2006 Bulletin 2006/15**

(21) Application number: **01951278.9**

(22) Date of filing: **06.07.2001**

(51) Int Cl.:
*A61K 9/20* (2006.01)          *A61K 9/14* (2006.01)

(86) International application number:
**PCT/CA2001/000993**

(87) International publication number:
**WO 2002/003962 (17.01.2002 Gazette 2002/03)**

(54) **DRUG DELIVERY SYSTEM FOR POORLY WATER SOLUBLE DRUGS**

ARZNEISTOFFABGABESYSTEM FÜR WASSER SCHWERLÖSLICHE ARZNEIMITTEL

SYSTEME D'APPORT DE MEDICAMENTS FAIBLEMENT HYDROSOLUBLES

(84) Designated Contracting States:
**AT BE CH CY DE DK ES FI FR GB GR IE IT LI LU
MC NL PT SE TR**

(30) Priority: **07.07.2000 US 216627 P**
        **21.11.2000 US 252389 P**

(43) Date of publication of application:
**09.04.2003 Bulletin 2003/15**

(73) Proprietor: **Kemestrie Inc.
Sherbrooke,
Québec J1L 2P4 (CA)**

(72) Inventors:
  • **CHORNET, Esteban
    Sherbrooke, Quebec J1L 1H3 (CA)**
  • **ISHIZAWA, Claudia
    Sherbrooke, Quebec J1K 2R6 (CA)**
  • **DUMITRIU, Severian
    Sherbrooke, Quebec J1L 1B8 (CA)**

(74) Representative: **Keller, Günter et al
Lederer & Keller
Patentanwälte
Prinzregentenstrasse 16
80538 München (DE)**

(56) References cited:
  **WO-A-00/04086          US-A- 5 620 706**

• **DUMITRIU, S. ET AL: "Chitosan - based hydrogels
as biomaterials." BOOK OF ABSTRACTS, 213TH
ACS NATIONAL MEETING, SAN FRANCISCO,
APRIL 13-17 (1997), CELL-128 PUBLISHER:
AMERICAN CHEMICAL SOCIETY,
WASHINGTON, D. C. , XP001053515**
• **DUMITRIU, S. ET AL: "Polyionic hydrogels as
support for controlled release of nifedipine"
PROC. INT. SYMP. CONTROLLED RELEASE
BIOACT. MATER. (2000), 27TH, 455-456 , 6 July
2000 (2000-07-06), XP002189752**
• **SHIRAISHI S ET AL: "CONTROLLED RELEASE
OF INDOMETHACIN BY CHITOSAN-
POLYELECTROLYTE COMPLEX: OPTIMIZATION
AND IN VIVO/IN VITRO EVALUATION" JOURNAL
OF CONTROLLED RELEASE, ELSEVIER
SCIENCE PUBLISHERS B.V. AMSTERDAM, NL,
vol. 25, no. 3, 1 June 1993 (1993-06-01), pages
217-225, XP000369908 ISSN: 0168-3659**
• **WATANABE KAZUNORI ET AL: "Investigation on
rectal absorption of indomethacin from
sustained-release hydrogel suppositories
prepared with water-soluble dietary fibers,
xanthan gum and locust bean gum."
BIOLOGICAL & PHARMACEUTICAL BULLETIN,
vol. 16, no. 4, 1993, pages 391-394, XP001053536
ISSN: 0918-6158**
• **K PARFITT (ED.): "Martindale. The complete drug
reference." , PHARMACEUTICAL PRESS ,
TAUNTON, MASSACHUSETTS XP002190167 *
Thirty-second edition. Pages viii and 1358. page
VIII, left-hand column, line 40 - line 49 page 1358,
left-hand column, line 42 - line 48**

EP 1 299 089 B1

**Description**

## FIELD OF THE INVENTION

The present invention relates to a method for modifying the solubilization rates of poorly water soluble drugs, using a chitosan-xanthan hydrogel. In addition, the present invention relates to a process for the preparation of chitosan-xanthane hydrogels as well as for the preparation of hydrogels comprising a poorly water soluble drug.

## BACKGROUND OF THE INVENTION

Drug solubility has been a common limitation in the development of new drug formulations. This may not be surprising given that more than a third of the drugs listed in the *United States Pharmacopoeia* are either poorly soluble or insoluble in water.[1] Additionally, it is well known that for many drugs the rate-limiting step for the absorption within the gastrointestinal tract, is its dissolution.[2]

In order to enhance the dissolution rate of poorly soluble drugs and increase their bioavailability, several techniques have been developed. A common option is the improvement of the solubility through specific formulation approaches.[3] One such approach is to prepare the drug in an amorphous form, *i.e.,* grinding the drug in the presence of certain additives such as porous powders.[4] Amorphous materials, however, are thermodynamically unstable and tend to revert to the crystalline form on storage.[5] Other techniques are based on particle size reduction, which is intended to increase the contact surface between the drug and the solvent.[1] An inadequate control of the particle size can produce variations in the solubilization rate due to agglomeration. In a few cases, a broad distribution of the particle size may have side effects such as gastric bleeding and nausea. Yet another approach for increasing drug dissolution rates involves the incorporation of surfactants and wetting agents or the formation of solid drug dispersions in water-soluble carriers.[6]

Hydrogels are three-dimensional structures capable of retaining large amounts of water. They can be formed by the interaction of oppositely-charged ionic polymers. Some natural poly-ions such as chitosan, carboxymethylcellulose, alginic acid, pectin and xanthane have been used to prepare hydrogels, within which bio-active materials and enzymes have been introduced.[7-10]

S. Damitriu describes in Proceed. Int'l Symp. Control. Rel. Bioact. Mater, 27 (2000) Controller Release Society, Inc., pages 455-456 polyionic hydrogels for controlled release of nifedipine. The molecular weight of chitosan in these hydrogels varies from 125,000 to 800,000 Da.

There remains a need to develop a method capable of improving the solubilization rates of poorly water soluble drugs.

## OBJECTS OF THE INVENTION

An object of the present invention is therefore to provide a chitosan-xanthane hydrogel for use as a system capable of modifying the solubilization rates of poorly water soluble drugs. A further object of the present invention is to disclose a process for the preparation of such a chitosan-xanthane hydrogel as well as a process for the preparation of a such a chitosan-xanthane hydrogel comprising a poorly water soluble drug.

## SUMMARY OF THE INVENTION

Generally, in accordance with the present invention, there is provided a method to modify the solubilization rates of poorly water soluble drugs. This modification is achieved by the inclusion of the drug in a hydrophilic matrix provided by the xanthane-chitosan hydrogel. The amount of drug incorporated in the hydrogel may attain a proportion of up to 50% (w/w), depending on the amount of drug added during the preparation. The average efficiency of drug inclusion ranges typically from 60 to 90%. The dissolution behavior of the drug included in the hydrogel is dependent on the hydrogel structure and is mainly a function of the chitosan characteristics, that is, the molecular weight (MW) and the degree of acetylation (DA).

In accordance with the present invention, there is provided a method for preparing a novel polymeric matrix or hydrogel containing a poorly water soluble drug, capable of modifying the dissolution behavior of poorly water soluble drugs, this method comprising the steps of dispersing a drug and forming a gel.

More specifically, in accordance with the present invention, there is provided a system behaving independently of the pH of the dissolution medium as well as providing for pH-sensitive matrices that can be prepared by selecting the proper characteristics of the xanthane and chitosan raw materials used to prepare the hydrogel.

Compositions comprising a poorly water soluble drug, chitosan and xanthane are further objects of the present invention.

Four drugs, that is, fenofibrate, ursodeoxycholic acid, nifedipine and indomethacin, are used as models of poorly water soluble drugs to be incorporated into the xanthane-chitosan hydrogel in an effort to improve their solubilization rate.

In accordance with the present invention, there is provided a drug delivery system comprising a chitosan-xanthane hydrogel which includes in its matrix a poorly water soluble drug which upon swelling of the hydrogel in an aqueous medium becomes at least partially solubilized and releasable therefrom.

In accordance with the present invention, there is provided a method for preparing a poorly water soluble drug delivery system comprising dissolving the poorly water soluble drug in an appropriate solvent to form a solution, adding the solution to a xanthane solution to form a dispersion, adding this dispersion to a chitosane solution and recuperating the resulting hydrogel.

In accordance with the present invention, there is also provided a method for modifying the solubilization rate of a poorly water soluble drug, comprising the step of including the poorly water soluble drug in a hydrogel composed of a chitosan-xanthane microstructure governing the solubilization rate.

As used herein, the terminology "poorly water soluble drug" refers to a drug requiring more than 10 000 mL to dissolve 1 g of the drug.

As used herein, the terminology "about" refers to a +/- 5% variation from the nominal value. Although not mentioned everywhere, it is to be understood that such a variation is always included in any given value herein below.

As used herein, the terminology "slow-release", well known in the art, refers to a release of <50% of a drug content in 15 minutes.

As used herein, the terminology "fast-release", well known in the art, refers to a release of greater or equal to 50% of a drug content in 15 minutes.

## BRIEF DESCRIPTION OF THE DRAWINGS

In the appended drawings:

Figure 1     Scanning electron microscopy images of the (a) external surface (40 000X) and (b) internal surface (60 000X) of typical hydrogels.

Figure 2     Dissolution profiles of slow-release rate fenofibrate system and of free pure fenofibrate. Fenofibrate content: 40%; Chitosan (MW: 1100000 Da; DA: 23%). Dissolution medium: 800 mL; Rotating speed: 55-60 rpm.

Figure 3     Scanning electron microscopy images of the hydrogel and the hydrogel containing 35% fenofibrate:

(a) hydrogel (2 500X)
(b) hydrogel containing fenofibrate (2 500X)
(c) hydrogel (5 000X)
(d) hydrogel containing fenofibrate (5 000X)

Figure 4     Dissolution profile of a rapid release fenofibrate system and of free pure fenofibrate. Fenofibrate content: 40%; Chitosan (MW: 800 000 Da; DA: 25%). Dissolution medium: 800 mL; Rotating speed: 55-60 rpm.

Figure 5     Dissolution profile of an instant-release fenofibrate system and of free pure fenofibrate. Fenofibrate content: 28%; Chitosan (MW: 540 000 Da; DA: 23%). Dissolution medium: 800 mL; Rotating speed: 55-60 rpm.

Figure 6     Dissolution profiles of a pH-sensitive fenofibrate system and of free pure fenofibrate. Fenofibrate content: 28%; Chitosan (MW: 800 000 Da; DA: 18%). Dissolution medium: 800 mL; Rotating speed: 55-60 rpm.

Figure 7     Dissolution profiles of systems with various fenofibrate contents and of free fenofibrate. Chitosan (MW: 540 000 Da; DA: 23%). Dissolution medium: 800 mL of sodium lauryl sulfate 0.025N; Rotating speed: 55-60 rpm.

Figure 8     Dissolution profiles of ursodeoxycholic acid systems and of free pure ursodeoxycholic acid. Dissolution medium: pH 6.2 potassium phosphate buffer solution; Rotating speed: 55-60 rpm.

Figure 9     Dissolution profiles of a slow-release nifedipine system and of free pure nifedipine. Nifedipine content: 35%; Chitosan (MW: 1 100 000 Da; DA: 23%); Dissolution medium: 800 ml; Rotating speed: 53-55 rpm.

Figure 10     Dissolution profiles of a rapid-release nifedipine system and of free pure nifedipine. Nifedipine content:

30%; Chitosan (MW: 800 000 Da; DA: 25%); Dissolution medium: 800 ml; Rotating speed: 53-55 rpm.

Figure 11    Dissolution profiles of nifedipine systems and of free pure nifedipine in a continuous flow cell apparatus. Dissolution medium: 800 mL of sodium lauryl sulfate 0.01N; Flow rate: 6.19 mL/min.

Figure 12    Dissolution profiles of indomethacin systems and of free pure indomethacin. Dissolution medium: pH 6.2 potassium phosphate buffer solution; Rotating speed: 73-75 rpm.

## DETAILED DESCRIPTION OF THE INVENTION

The present invention describes a method for preparing "intelligent" hydrogels, having the ability to modify and adapt the dissolution rate of poorly water soluble drugs to specific media. The method has been shown to be effective in enhancing the dissolution rate of poorly water soluble drugs.

One of the most important advantages of modifying the dissolution rates of poorly water soluble drugs *via* the use of hydrogels, is the ability to modify the particle size of the drug, the ability to modify its amorphous vs crystalline state and the ability to modify its dispersion in the hydrogel matrix without any chemical or mechanical manipulation ensuring that no modification to the chemical structure of the drug is introduced.

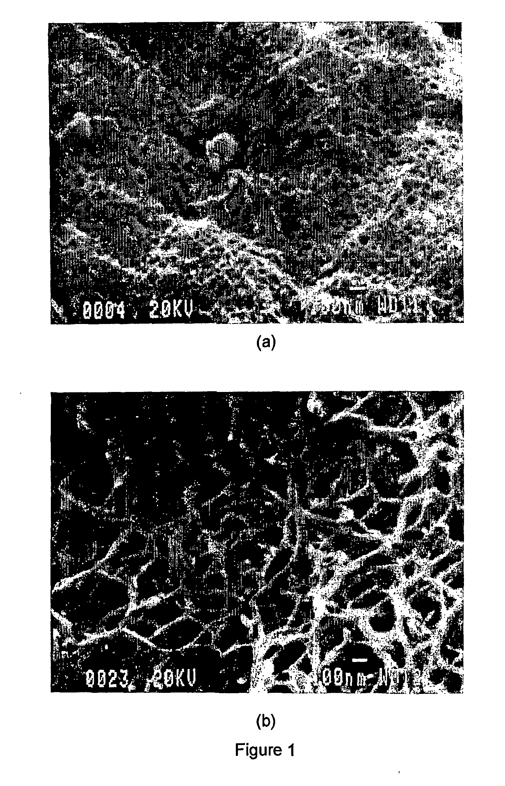

The hydrogel of the present invention is composed of two natural polymers, chitosan and xanthane. These two natural polymers comprise the raw materials for the matrix preparation. Scanning electron microscopy of the freeze-dried hydrogel revealed the presence of a porous and fibrillar structure, with pore sizes ranging from $10^{-7}$ to $10^{-6}$ m. The fibrils were shown to have a dimension of approximately $10^{-7}$ m. Scanning electron microscopy images of the external surface and of the internal section are shown in Figure 1.

In the present invention, four drugs, fenofibrate, ursodeoxycholic acid, nifedipine and indomethacin have been used as poorly water soluble drug models.

**Brief description of the poorly water soluble drugs used in the present study.**

Fenofibrate

Fenofibrate, an ester derived from fibric acid, is a sparingly soluble drug used to reduce the plasma concentrations of cholesterol and of triglycerides.[11] Its solubility is not affected by the pH of the dissolution medium. Several studies have reported methods whose aim is the increase of the dissolution characteristics of finofibrate. Some of the reported methods involve dispersing the drug in polyethylene glycol (PEG) and polyvinylpyrrolidone (PVP), with the aim of forming essentially molecular dispersions.[12,13] Other methods disclose inclusion/complexation systems with cyclodextrins and yet other methods involve micronization using supercritical carbon dioxide.[14,15]

As is disclosed in the present invention, the incorporation of fenofibrate into a hydrogel has allowed for the modification of its solubility behavior. This modification is facilitated by the fact that the hydrogel delivers the drug *via* a process involving swelling-diffusion through the hydrogel matrix. Changes in the swelling-diffusion patterns account for the behavior of the different hydrogel formulations.

Ursodeoxycholic acid

A drug used for the dissolution of cholesterol gallstones. The compound has a low water solubility at pH values below 7.0. In order to improve the dissolution rates of ursodeoxycholic acid, a variety of techniques for preparing solid dispersions such as mixing, milling and solvent evaporation using water-soluble carriers such as polyethylene glycol, urea, mannitol as well as cellulose and starch derivatives, have been reported.[16-18]

Nifedipine

A poorly water soluble drug, used in the treatment of angina pectoris and hypertension. Due to its poor water solubility, its absorption is limited by its dissolution rate. Several methods aimed at enhancing the solubility of nifedipine have been investigated. Such methods include the formation of solid dispersions with polymers and the preparation of inclusion complexes with cyclodextrins.[19-23]

Indomethacin

An effective poorly water soluble non-steroidal antiinflammatory drug. Due to its poor water solubility, oral administration often results in gastric irritation. The enhancement and control of indomethacin solubility may avoid or decrease adverse side effects. Systems aimed at improving the solubility of indomethacin, such as the use of polystyrene microparticles, the use of new chitosan based excipients for tablets, the use of co-solvents and cyclodextrins, have recently been investigated. [24-27]

**Analytical methods used to quantify the drug concentration and characterize the hydrogel**

Fenofibrate, nifedipine and indomethacin spectroscopic quantification

The quantification of the drug content in the different hydrogel preparations or in *in vitro* dissolution tests is determined spectrophotomerically (UV-Vis Spectrophotometer). The fenofibrate concentration is determined at 289 nm, the nifedipine concentration at 340 nm and the indomethacin concentration at 320 nm. The observed absorbance data are transformed into concentration values, using a standard calibration curve which is obtained experimentally ($R^2$=0.999) in the corresponding medium.

HPLC quantification of Ursodeoxycholic acid

The quantification of the drug content in the different hydrogel preparations or in *in vitro* dissolution tests is determined by high performance liquid chromatography (HPLC). The separation is performed in a 5 $\mu$m Luna C-18(2) column (250 x 4.6 mm i.d.) eluted under isocratic conditions with a mobile phase composed of methanol-water-phosphoric acid (77:22.4:0.6 v/v/v). Analyses are performed at room temperature at a flow rate of 1.0 ml/min. and with an injection volume of 20 $\mu$L. Ursodeoxycholic acid is detected by a refractive index detector.

Drug content determination

The following method is also used to determine the fenofibrate, ursodeoxychlolic acid, nifedipine and indomethacin content in the hydrogels. An accurately weighed quantity of hydrogel is introduced in a centrifuge tube, and extracted with a specific solvent over a 1 hour period under stirring. Following the extraction period, the sample is centrifuged, filtered, suitably diluted and analyzed. Each determination is carried-out in duplicate.

Evaluation of the water uptake capacity of the hydrogel

100 mg of accurately weighed hydrogel are placed in a centrifuge tube containing 30 mL of water at room temperature. The tube is repeatedly turned upside down to thoroughly wet the hydrogel. After 2 hours the hydrogel is removed from the tube by centrifugation and decantation. The hydrogel is allowed to drain and is re-weighed. The increase in weight represents the weight of water taken-up by the hydrogel. The water uptake capacity ($\alpha$) is calculated as the ratio of the weight of absorbed water to the weight of dry hydrogel, as represented by Equation 1.

$$\text{Water uptake capacity [g/g]} = \alpha = \frac{W_{wet} - W_{dry}}{W_{dry}} \times 100$$

$$\text{Equation 1}$$

Determination of the drug dissolution rate from hydrogels

a) *In Vitro* Dissolution Test: Rotating Paddle Apparatus

The dissolution tests are conducted in a rotating paddle apparatus at 37˚C, and the rotating speed is set depending on drug tested. The dissolution tests are performed in different media, using volumes of 800 mL: a) potassium phosphate buffer (pH 7.4) containing 0.5% of Tween 80; b) hydrochloric acid buffer (pH 1.2) containing 0.5% of Tween 80; c) sodium lauryl sulfate solution (0.025 N); d) potassium phosphate buffer (pH 7.4); e) potassium phosphate buffer (pH 6.2). At specific time intervals following the test initiation (*i.e.,* insertion of the sample into the apparatus), aliquots (2 mL) are withdrawn from the test medium and immediately replaced with fresh medium in order to maintain the volume. The withdrawn samples, following suitable dilution, are either analyzed spectrophotometrically or by high performance liquid chromatography (HPLC), depending on the drug tested. Dissolution results are reported as the cumulative percentage of drug dissolved versus time.

b) *In Vitro* Dissolution Test: Continuous Flow Cell Apparatus

The dissolution tests are conducted in a continuous flow cell apparatus at 37°C, and the flow rate depends on drug tested. The dissolution tests are performed in volumes of 800 mL of a sodium lauryl sulfate solution (0.01N). At indicated times, aliquots (2 mL) are withdrawn and analyzed spectrophotometrically. The dissolution results are reported as cumulative amounts (mg) of drug dissolved versus time.

Scanning Electron Microscopy (SEM)

Sample surfaces are examined with a scanning electron microscope. For analyses, freeze-dried hydrogels are fixed on a SEM holder and coated with Au-Pd.

These drugs and methods of monitoring thereof, have been used to demonstrate that a chitosane-xanthane based hydrogel may be combined with a poorly water soluble drug in order to increase the solubility of the poorly water soluble drug. The kinetics of drug release may be modified by selecting the proper characteristics of chitosan (MW and DA) which govern the drug retention and release speed.

## **EXAMPLES**

Example 1: Fenofibrate slow-release rate dissolution system

A process for the preparation of a fenofibrate slow-release rate dissolution hydrogel is illustrated. The product is characterized by: a) fenofibrate content, b) water uptake capacity, c) *in vitro* dissolution tests and d) scanning electron microscopy.

In order to prepare a slow-release rate system, a chitosan with a high molecular weight is selected (MW: 1 100 000 Da; DA: 23%). The hydrogel is prepared following a two-step process. In a first step fenofibrate (2g) is dissolved in ethanol (100 mL), which is then added under vigorous stirring to an aqueous xanthane solution (300mL of a 0.65% (w/v)). At this point, a homogeneous dispersion of the drug is formed. The second step involves the hydrogel formation, which is achieved by adding the drug-xanthane dispersion to a high molecular weight aqueous chitosan solution (250mL of a 0.65% (w/v)). The mixture is stirred for 2h and then thoroughly washed with water. The final product, the dried gel, is obtained after freeze-drying.

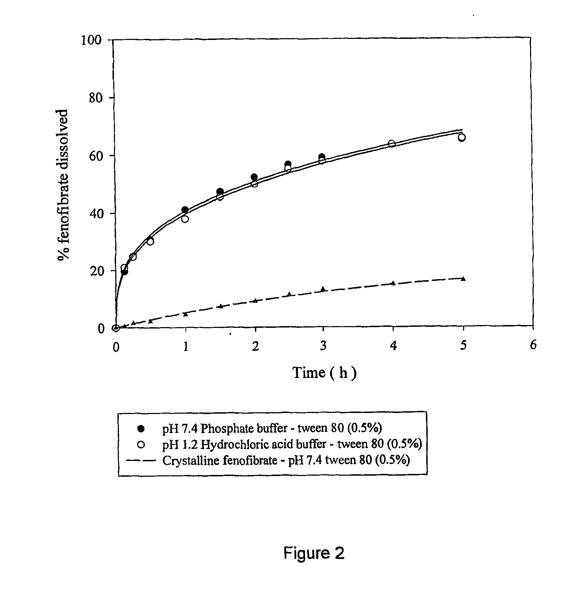

A fenofibrate content in the hydrogel of about 40% (w/w) was determined, following the procedure previously described and using ethanol as the extraction solvent. The water uptake capacity "$\alpha$" was ascertained as being 2700. Dissolution tests were performed in different dissolution media at 37°C, using samples comprising an equivalent of 20mg of fenofibrate. As shown in Figure 2, the results demonstrate a slow-release dissolution rate for fenofibrate, independent of pH.

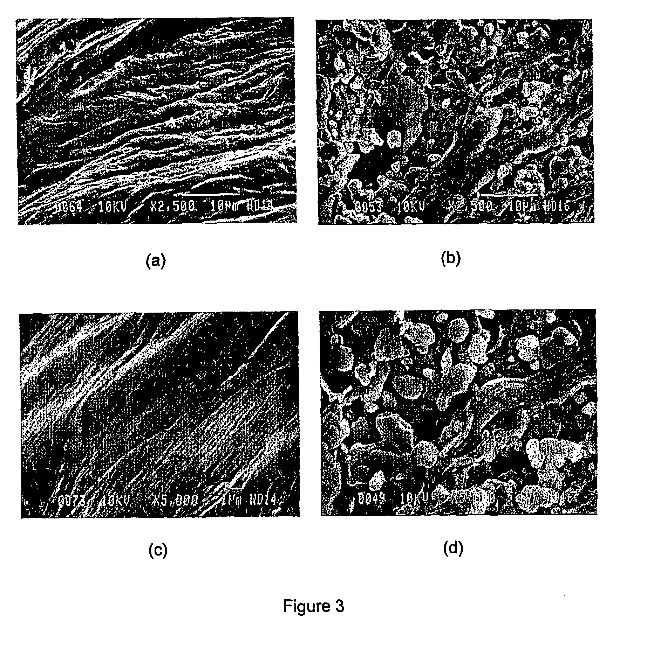

While fenofibrate is a crystalline drug, the freeze-dried hydrogel containing fenofibrate is a non-elastic powder displaying a white color. Surface analysis by scanning electron microscopy illustrated that incorporating the drug into the hydrogel, promotes the formation of drug microstructures with sizes in the order of microns. Images of the hydrogel and of the hydrogel containing fenofibrate, are presented in Figure 3.

Ref. Example 2: Fenofibrate rapid-release rate dissolution system

A process for the preparation of a fenofibrate rapid-release rate dissolution system is illustrated. The hydrogel is prepared in the same manner as described in Example 1, with the exception of the use of a medium to high molecular weight chitosan (MW: 800 000 Da; DA: 25%).

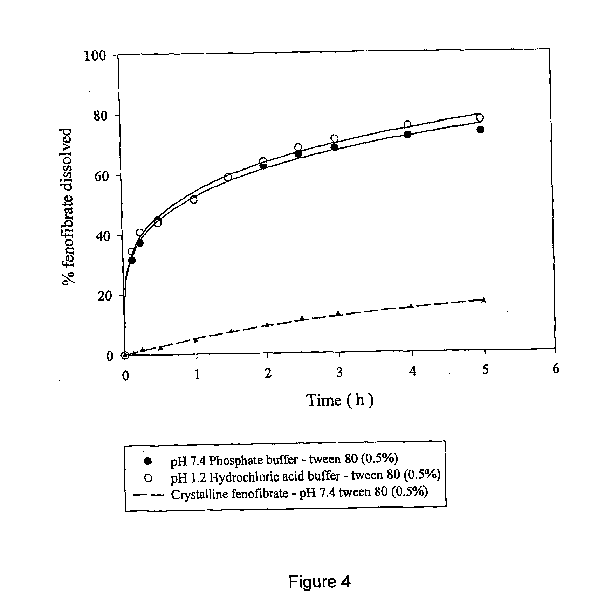

The fenofibrate content is about 40% (w/w) and the water uptake capacity ($\alpha$) is 2000. Dissolution tests were performed in different dissolution media at 37°C, with samples comprising an equivalent of 20mg of fenofibrate. As shown in Figure 4, the results demonstrate a rapid-release rate for fenofibrate, independent of pH.

Ref. Example 3: Fenofibrate instant-release system

A process for the preparation of an instant-release fenofibrate dissolution system is illustrated. The hydrogel is prepared in the same manner as described in Example 1, with the exception of the use of a medium molecular weight chitosan (MW: 540 000 Da; DA: 23%).

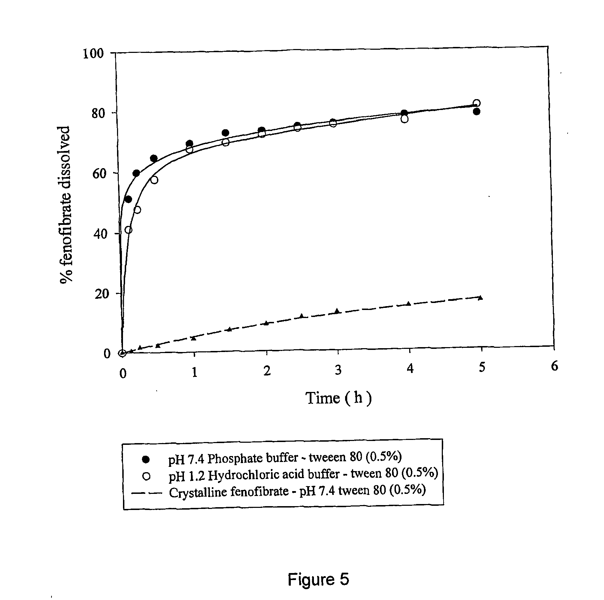

The fenofibrate content is about 28% (w/w) and the water uptake capacity ($\alpha$) is 1600. Dissolution tests were performed in different dissolution media at 37°C, with samples comprising an equivalent of 20mg of fenofibrate. As illustrated in Figure 5, the results demonstrate an essentially instant release of fenofibrate, independent of pH.

Ref. Example 4: Fenofibrate pH-sensitive dissolution system

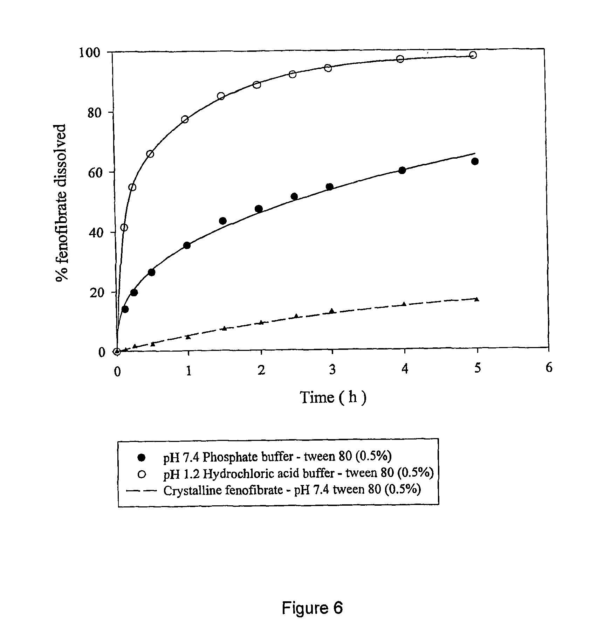

A process for the preparation of a pH-sensitive fenofibrate dissolution system is illustrated. The hydrogel is prepared in the same manner as described in Example 1, with the exception of the use of a medium-high molecular weight chitosan having a low degree of acetylation (MW: 800 000 Da; DA: 18%) and with an agitation time that is reduced to 45 minutes.
The fenofibrate content is about 28% (w/w) and the water uptake capacity ($\alpha$) is 2000. Dissolution tests were performed in different dissolution media at 37˚C, with samples comprising an equivalent of 20mg of fenofibrate. As illustrated in Figure 6, the results demonstrate that the system has a low dissolution rate at pH 7.4 while becoming a rapid-dissolution rate system at pH 1.2.

Ref. Example 5: Varying drug content

A process for the preparation of hydrogels with various fenofibrate contents is illustrated. The hydrogels are prepared in the same manner as described in Example 1, with the exception that the amount of fenofibrate added during the preparations is altered depending on the required composition. A medium molecular weight chitosan (MW: 540 000 Da; DA: 23%) is used in the preparation of the hydrogels. The fenofibrate content incorporated in the different samples is summarized in Table 1.

**Table 1**: Fenofibrate content for different samples

| Sample | Fenofibrate (g) | Drug content % (w/w) |
|---|---|---|
| 1 | 1.0 | 21.2 |
| 2 | 1.5 | 30.6 |
| 3 | 2.0 | 40.2 |

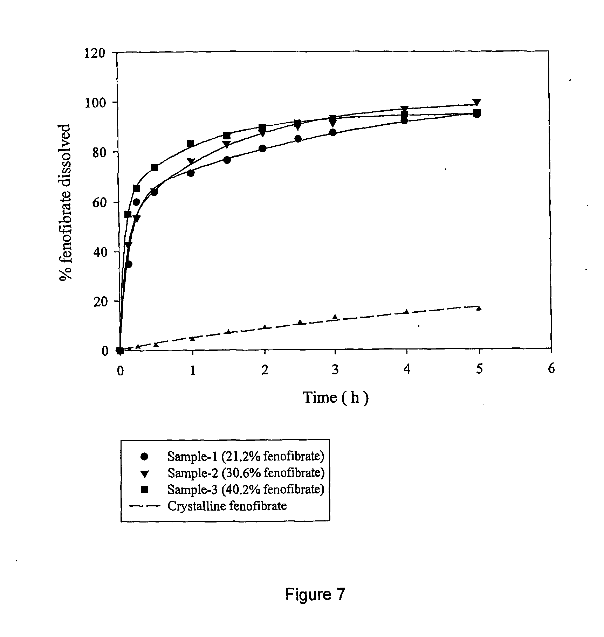

The drug content in the samples is varied between 20 and 40% (w/w). Dissolution tests were performed in a sodium lauryl sulfate solution (0.025N), with samples comprising an equivalent of 20mg of fenofibrate. As illustrated in Figure 7, the results demonstrate that the dissolution rate is independent of the drug content.

Example 6: Ursodeoxycholic acid slow-release rate dissolution system

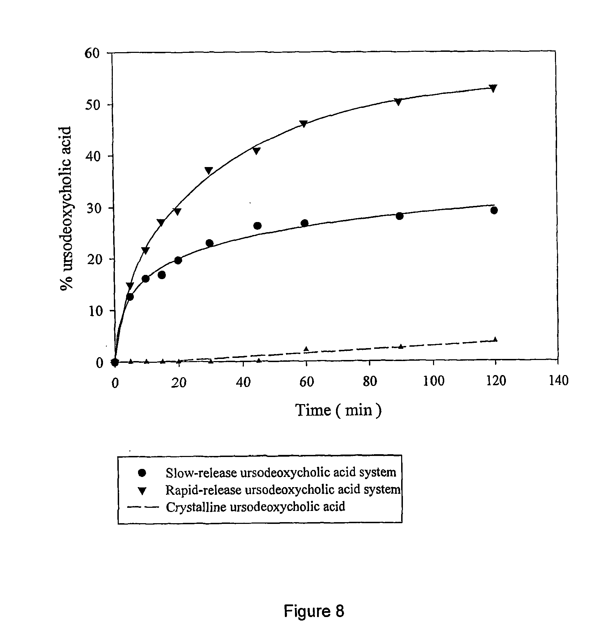

A process for the preparation of an ursode,oxychotic acid slow-release rate dissolution system is illustrated. The hydrogels are prepared in the same manner as described in Example 1, with the exception of the use of ursodeoxycholic acid as the drug.
The ursodeoxycholic acid content is about 30% (w/w) and the water uptake capacity ($\alpha$) is 2800. Dissolution tests were performed in a phosphate buffer solution (pH 6.2) at 37˚C, with samples comprising an equivalent of 150mg of ursodeoxycholic acid. As shown in Figure 8 the results demonstrate a slow-release dissolution rate for ursodeoxycholic acid.

Ref. Example 7: Ursodeoxycholic acid rapid-release rate dissolution system

A process for the preparation of an ursodeoxycholic acid rapid-release rate dissolution system is illustrated. The hydrogel is prepared in the same manner as described in Example 2, with the exception of the use of ursodeoxycholic acid as the drug.
The ursodeoxycholic acid content is about 20% (w/w) and the water uptake capacity ($\alpha$) is 2000. Dissolution tests were performed in a phosphate buffer solution (pH 6.2) at 37˚C, with samples comprising an equivalent of 150mg of ursodeoxycholic acid. As shown in Figure 8 the results demonstrate a rapid-release dissolution rate for ursodeoxycholic acid.

Example 8: Nifedipine slow-release rate dissolution system*

A process for the preparation of a nifedipine slow-release rate dissolution system is illustrated. The hydrogels are prepared in the same manner as described in Example 1, with the exception of the use of nifedipine as the drug.

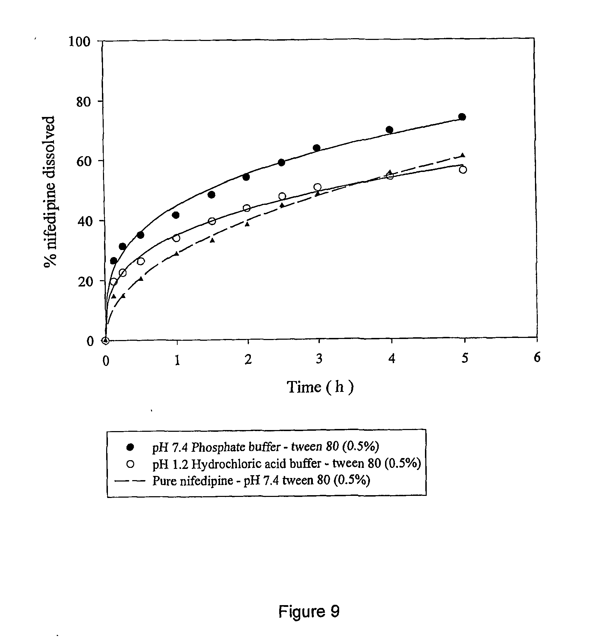

The nifedipine content is about 35% (w/w) and the water uptake capacity ($\alpha$) is 2800. Dissolution tests were performed in two different dissolution media at 37˚C, with samples comprising an equivalent of 30mg of nifedipine. As shown in Figure 9, the results demonstrate a slow-release dissolution rate for nifedipine.
*Preparation and tests with nifedipine were carried out under light-protected conditions to prevent the photodecomposition of nifedipine.

Ref .Example 9: Nifedipine rapid-release rate dissolution system*

A process for the preparation of a nifedipine rapid-release rate dissolution system is illustrated. The hydrogel is prepared in the same manner as described in Example 2, with the exception of the use of nifedipine as the drug.

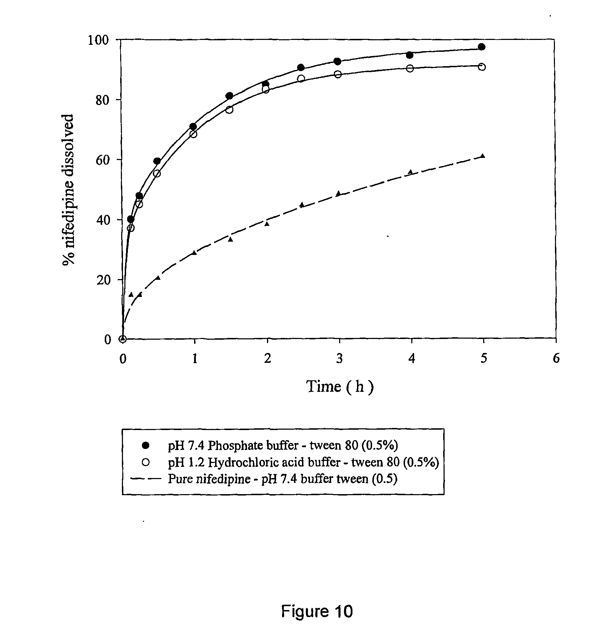

The nifedipine content is about 30% (w/w) and the water uptake capacity ($\alpha$) is 2000. Dissolution tests were performed in two different dissolution media at 37˚C, with samples comprising an equivalent of 30mg of nifedipine. As shown in Figure 10, the results demonstrate a rapid-release dissolution rate for nifedipine.
*Preparation and tests with nifedipine were carried out under light-protected conditions to prevent the photodecomposition of nifedipine.

Example 10: Nifedipine hydrogels tested in a continuous flow cell apparatus

Dissolution tests of nifedipine hydrogels in a continuous flow cell apparatus are illustrated. The nifedipine content in the tested samples varied between 24 to 40% (w/w).

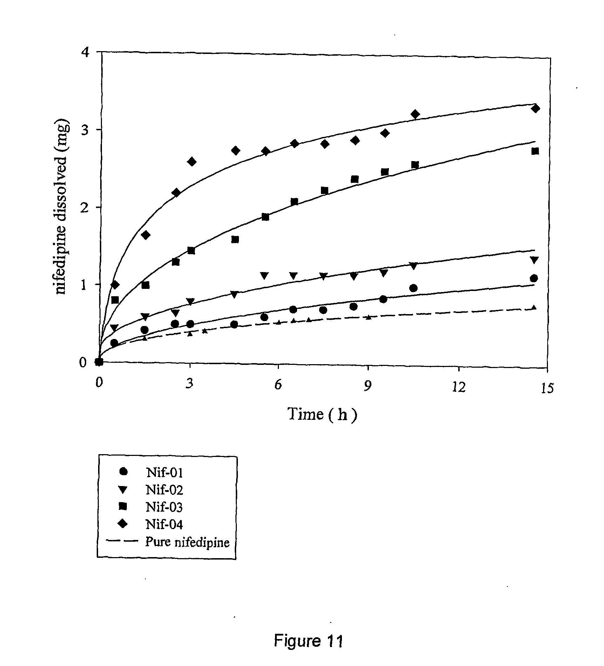

Dissolution tests were performed in a sodium lauryl sulfate solution (0.01 N) at 37˚C with a flow-rate of 6.19 mL/min, with samples comprising an equivalent of 40mg of nifedipine. As illustrated in Figure 11, the dissolution results are reported as the cumulative amount (mg) of drug dissolved versus time.
*Preparation and tests with nifedipine were carried out under light-protected conditions to prevent the photodecomposition of nifedipine.

Example 11: Indomethacin slow-release rate dissolution system

A process for the preparation of an indomethacin slow-release rate dissolution system is illustrated. The hydrogel is prepared in the same manner as described in Example 1, with the exception of the use of indomethacin as the drug.

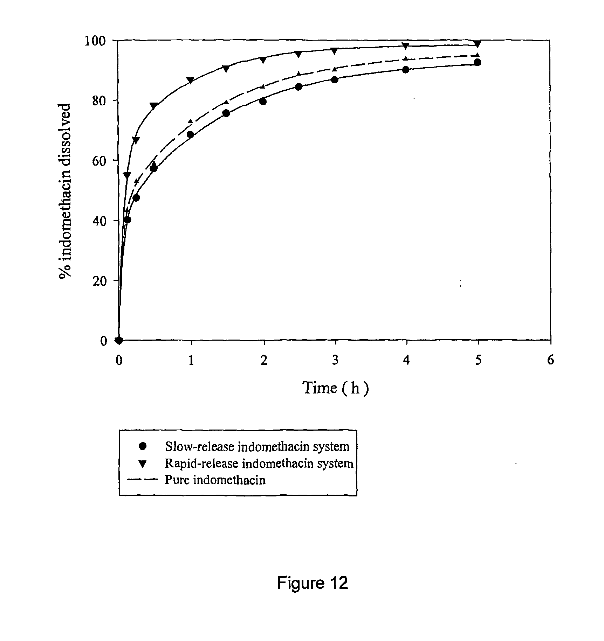

The indomethacin content is about 45% (w/w) and the water uptake capacity ($\alpha$) is 2800. Dissolution tests were performed in a phosphate buffer solution (pH 6.2) at 37˚C, with samples comprising an equivalent of 30mg of indomethacin. As shown in Figure 12 the results demonstrate a slow-release dissolution rate for indomethacin.

Ref. Example 12: Indomethacin rapid-release rate dissolution system

A process for the preparation of an indomethacin rapid-release rate dissolution system is illustrated. The hydrogel is prepared in the same manner as described in Example 2, with the exception of the use of indomethacin as the drug.

The indomethacin content is about 35% (w/w) and the water uptake capacity ($\alpha$) is 2000. Dissolution tests were performed in a phosphate buffer solution (pH 6.2) at 37˚C, with samples comprising an equivalent of 30mg of indomethacin. As shown in Figure 12 the results demonstrate a rapid-release dissolution rate for indomethacin.

The upper and lower limits as to the make-up of the hydrogels, prepared in accordance to the present invention, are reported in Table 2.

**Table 2:** Upper and lower limits of the hydrogel compositions.

|  | Lower Limit | Upper Limit |
|---|---|---|
| Chitosan (%) | 18 | 35 |
| Xanthane (%) | 32 | 55 |
| Poorly water soluble drug (%) | 10 | 50 |

The hydrogels of the present invention will contain approximately about 18 to 35% by weight of chitosane, about 32 to 55% by weight of xanthane and about 10 to 50% by weight of a poorly water soluble drug. The degree of acetylation (DA) of chitosan typically ranges from about 10 to about 30%.

A high molecular weight chitosan is typically selected from a field ranging from about 900 000 Da to about 1 200 000 Da.

## REFERENCES

1. S. Pace, G. W. Pace, 1. Parikh, A. K. Mishra; Novel injectable formulations of insoluble drugs. *Pharm. Tech.* March (1999) 116-132.

2. D. Q. M. Craig, P. G. Royall, V. L. Kett, M. L. Hopton; The relevance of the amorphous state to pharmaceutical dosage forms: glassy drugs and freeze dried systems. *Int. J. Pharm.* 179 (1999) 179-207.

3. L. Leuner, J. Dressman; Improving drug solubility for oral delivery using solid dispersions. *Eur. J. Pharm. Biopharm.* 50 (2000) 47-60.

4. E. Yonemochi, M. Kojima, A. Nakatsuji, S. Okonogi, T. Oguchi, Y. Nakai, K. Yamamoto; Thermal behavior of methyl *p*-hydroxybenzoate in controlled-pore glass solid dispersion. *J. Colloid Interphase Sci.* 173 (1995) 186-191.

5. A. T. Florence, D. Atwood. *Physiochemical principles of pharmacy,* 3rd Edition, Creative Print & Design, Wales (1998).

6. D. E. Storey; The role of dissolution testing in the design of immediate release dosage forms. *Drug Information Journal* 30 (1996) 1039-1044.

7. S. Dumitriu, P. Magny, D. Montane, P. F. Vidal, E. Chornet; Polyionic hydrogels obtained by complexation between xanthan and chitosan. Their properties as support for enzyme immobilization. *J. Bioactive and Compatible Polymers* 9 (1994) 184-209.

8. S. Dumitriu, E. Chornet, P. F. Vidal; Polyionic insoluble hydrogels comprising xanthan. *US Patent 5620706,* Apr. 15, 1997.

9. S. Dumitriu, E. Chornet; Inclusion and release of proteins from polysaccharide-based polyion complexes. *Adv. Drug Deliv. Rev.* 31 (1998) 223-246.

10. S. Dumitriu, P. F. Vidal, E. Chornet; Hydrogel based on polysaccharides. *Polysaccharides on Medical Applications,* S. Dumitriu (Ed.), Marcel Dekker, New York (1996) 125-241.

11. J. A. Balfour, D. McTavish, R. C. Heel; Fenofibrate. A review of its pharmacodynamic properties and therapeutic use in dyslipidaemia. *Drugs* 40 (1990) 260-290.

12. M.-T. Sheu, C.-M. Yeh, T. D. Sokoloski; Characterization and dissolution of fenofibrate solid dispersion systems. *Int. J. Pharm.* 103 (1994) 137-146.

13. G. F. Palmieri, 1. Antonini, S. Martelli; Characterization and dissolution studies of PEG 4000/fenofibrate solid dispersions. *STP Pharma Sciences* 6 (1996) 188-194.

14. G. F. Palmieri, I. Antonini, S. Martelli; Inclusion complexation of fenofibrate with β-cyclodextrin and hydroxypropyl-β-cyclodextrin. Evaluation of interactions in solution and solid complex characterization. *STP Pharma Sciences* 7 (1997) 174-181.

15. J. Kerč, S. Srčič, Ž. Knez, P. Senčar-Božič; Micronization of drugs using supercritical carbon dioxide. *Int. J. Pharm.* 182 (1999) 33-39.

16. P. Giunchedi, S. Scalia, L. Maggi, U. Conte; Ursodeoxycholic acid: improvement of dissolution behaviour and its HPLC determination. *Int. J. Pharm.* 130 (1996) 41-47.

17. S. Higginbottom, C. B. Mallinson, S. J. Burns, D. Attwood, S. G. Barnwell; Ursodeoxycholic acid: effects of formulation on "In Vitro" dissolution. *Int. J. Pharm.* 109 (1994) 173-180.

18. S. Okonogi, E. Yonemochi, T. Oguchi, S. Puttipipatkhachorn, K. Yamamoto; Enhaced dissolution of ursodeoxycholic acid from the solid dispersion. *Drug Development and Industrial Pharmacy* 23 (1997) 1115-1121.

19. H. Suzuki, H. Sunada; Influence of water-soluble polymers on the dissolution of nifedipine solid dispersions with combined carriers. *Chem. Pharm. Bull.* 46 (1998) 482-487.

20. M. Guyot, F. Fawaz; Nifedipine loaded-polymeric microspheres: preparation and physical characteristics. *Int. J. Pharm.* 175 (1998) 61-74.

21. A. Portero, C. Remuñan- López, J. L. Vila-Jato; Effect of chitosan and chitosan glutamate enhancing the dissolution properties of the poorly water soluble drug nifedipine. *Int. J. Pharm.* 175 (1998) 75-84.

22. K. P. R. Chowdary, G. Girija Sankar; Eudragit microcapsules of nifedipine and its dispersions in HPMC-MCC: physicochemical characterization and drug release studies. *Drug Dev. Ind. Pharm.* 23 (1997) 325-330.

23. J. Filipović-Grčić, M. Bećirević-Laćan, N. Škalko, 1. Jalšenjak; Chitosan microspheres of nifedipine and nifedipine-cyclodextrin inclusion complexes. *Int. J. Pharm.* 135 (1996) 183-190.

24. S. Tamilvanan, B. Sa; Studies on in vitro release behavior of indomethacin-loaded polystyrene microparticles. *Int. J. Pharm.* 201 (2000) 187-197.

25. K. Aiedeh, I. Orienti, V. Bertasi, V. Zecchi; Chitosan and chitosan linked to triethylene glycol glutarate or betain as tabletting excipients for the sustained release of indomethacin. *S.T.P. Pharma Sci.* 8 (1998) 291-296.

26. M. Becirevic-Lacan; Inclusion complexation of indomethacin with cyclodextrins in solution and in the solid state. *S.T.P. Pharma Sci.* 4 (1994) 282-286.

27. A. M. Shawesh, S. Kallioinen, L. Hellén, J. Yliruusi; Evaluation of indomethacin solubility in different co-solvents and effect of enhancers on the solubility. *Pharmazie* 53 (1998) 567-569.

**Claims**

1. A drug delivery system for delivering at least one active ingredient having a solubility lower than 1 gram per 10 liters of aqueous medium, said drug delivery system consisting essentially of a chitosan-xanthane hydrogel having a 18 to 35 wt% content of chitosan and a 32 to 55 wt% content of xanthane with the remainder being the active ingredient (s), which upon swelling of said hydrogel in an aqueous medium becomes at least partially solubilized and releasable therefrom, said drug delivery system being **characterised by** a chitosan component of high molecular weight of between 900 to 1200 kilo Daltons.

2. The drug delivery system of claim 1, wherein said chitosan possesses a degree of acetylation ranging from 10 to 30%.

3. The drug delivery system of claim 1, wherein the amount of said drug is between 10 and 50 wt%.

4. The drug delivery system of any one of claims 1 to 3 **characterised in that** the active ingredient(s) is selected from the group consisting of fenofibrate, ursodeoxycholic acid, nifedipine and indomethacin.

5. The drug delivery system of claim 4 **characterised in that** the active ingredient is fenofibrate.

6. The drug delivery system of claim 4 **characterised in that** the active ingredient is ursodeoxycholic acid.

7. The drug delivery system of claim 4 **characterised in that** the active ingredient is nifedipine.

8. The drug delivery system of claim 4 **characterised in that** the active ingredient is indomethacin.

9. A method for preparing the drug delivery system of claim 1, comprising the steps of:

   a) dissolving the active ingredient(s) in ethanol to form of a solution;
   b) adding said solution to an aqueous xanthane solution having 0.65% wt/volume of xanthane so as to form a dispersion of the active ingredient(s) in the xanthane solution;
   c) adding said dispersion to an aqueous chitosan solution wherein the chitosan has a degree of acetylation of between 10 to 30% and a molecular weight of between 900 and 1200 kilo Daltons so as to form a hydrogel loaded with the active ingredient(s), and
   d) recuperating the hydrogel thus formed.

**Patentansprüche**

1. Wirkstoffabgabesystem zur Abgabe mindestens eines aktiven Inhaltsstoffs mit einer Löslichkeit von weniger als 1 g/10 l wässrigem Medium, wobei das Wirkstoffabgabesystem im Wesentlichen aus einem Chitosan-Xanthan-Hydrogel besteht mit einem Gehalt an Chitosan von 18 bis 35 Gew.-% und einem Gehalt an Xanthan von 32 bis 55 Gew.-%, wobei der Rest der aktive Inhaltsstoff/die aktiven Inhaltsstoffe ist/sind, der beim Quellen des Hydrogels in einem wässrigen Medium mindestens teilweise solubilisiert und daraus freisetzbar wird, wobei das Wirkstoffabgabesystem durch eine Chitosankomponente mit einem hohen Molekulargewicht zwischen 900 und 1.200 kD **gekennzeichnet** ist.

2. Wirkstoffabgabesystem nach Anspruch 1, wobei das Chitosan einen Acetylierungsgrad im Bereich von 10 bis 30% besitzt.

3. Wirkstoffabgabesystem nach Anspruch 1, wobei die Menge des Wirkstoffs zwischen 10 und 50 Gew.-% liegt.

4. Wirkstoffabgabesystem nach einem der Ansprüche 1 bis 3, **dadurch gekennzeichnet, dass** der aktive Inhaltsstoff/die aktiven Inhaltsstoffe aus der Gruppe ausgewählt sind, die aus Fenofibrate, Ursodesoxycholinsäure, Nifedipine und Indomethacin besteht.

5. Wirkstoffabgabesystem nach Anspruch 4, **dadurch gekennzeichnet, dass** der aktive Inhaltsstoff Fenofibrate ist.

6. Wirkstoffabgabesystem nach Anspruch 4, **dadurch gekennzeichnet, dass** der aktive Inhaltsstoff Ursodesoxycholinsäure ist.

7. Wirkstoffabgabesystem nach Anspruch 4, **dadurch gekennzeichnet, dass** der aktive Inhaltsstoff Nifedipine ist.

8. Wirkstoffabgabesystem nach Anspruch 4, **dadurch gekennzeichnet, dass** der aktive Inhaltsstoff Indomethacin ist.

9. Verfahren zur Herstellung des Wirkstoffabgabesystems von Anspruch 1, das die Stufen aufweist, dass

   a) der aktive Inhaltsstoff/die aktiven Inhaltsstoffe in Ethanol gelöst werden, um eine Lösung zu bilden;
   b) die Lösung zu einer wässrigen Xanthanlösung mit 0,65% Gewicht/Volumen Xanthan zugegeben wird, um eine Dispersion des aktiven Inhaltsstoffs/der aktiven Inhaltsstoffe in der Xanthanlösung zu bilden;
   c) die Dispersion zu einer wässrigen Chitosanlösung zugegeben wird, wobei das Chitosan einen Acetylierungsgrad zwischen 10 und 30% hat und ein Molekulargewicht zwischen 900 und 1.200 kD hat, um ein mit dem aktiven Inhaltsstoff/den aktiven Inhaltsstoffen beladenes Hydrogel zu bilden und
   d) das so gebildete Hydrogel gewonnen wird.


**Revendications**

1. Système de délivrance de médicaments destiné à délivrer au moins un ingrédient actif ayant une solubilité inférieure à 1 gramme pour 10 litres de milieu aqueux, ledit système de délivrance de médicaments étant essentiellement constitué par un hydrogel à base de chitosane-xanthane ayant une teneur de 18 à 35 % en poids de chitosane et une teneur de 32 à 55 % en poids de xanthane, le reste étant l'ingrédient (les ingrédients) actif(s), qui sous l'effet du gonflement dudit hydrogel dans un milieu aqueux se solubilise au moins partiellement et peut se libérer de celui-ci, ledit système de délivrance de médicaments étant **caractérisé par** un composant chitosane de masse moléculaire élevée comprise entre 900 et 1 200 kilodaltons.

2. Système de délivrance de médicaments selon la revendication 1, dans lequel ledit chitosane possède un degré d'acétylation dans la plage de 10 à 30 %.

3. Système de délivrance de médicaments selon la revendication 1, dans lequel la quantité dudit médicament est comprise entre 10 et 50 % en poids.

4. Système de délivrance de médicaments selon l'une quelconque des revendications 1 à 3, **caractérisé en ce que** l'ingrédient (les ingrédients) actif(s) est (sont) choisi(s) dans le groupe constitué par le fénofibrate, l'acide ursodésoxycholique, la nifédipine et l'indométhacine.

**5.** Système de délivrance de médicaments selon la revendication 4, **caractérisé en ce que** l'ingrédient actif est le fénofibrate.

**6.** Système de délivrance de médicaments selon la revendication 4, **caractérisé en ce que** l'ingrédient actif est l'acide ursodésoxycholique.

**7.** Système de délivrance de médicaments selon la revendication 4, **caractérisé en ce que** l'ingrédient actif est la nifédipine.

**8.** Système de délivrance de médicaments selon la revendication 4, **caractérisé en ce que** l'ingrédient actif est l'indométhacine.

**9.** Procédé de préparation du système de délivrance de médicaments selon la revendication 1, comprenant les étapes consistant à :

a) dissoudre l'ingrédient (les ingrédients) actif(s) dans de l'éthanol pour former une solution ;
b) ajouter ladite solution à une solution aqueuse de xanthane ayant 0,65 % pds/volume de xanthane de manière à former une dispersion de l'ingrédient (des ingrédients) actif(s) dans la solution de xanthane ;
c) ajouter ladite dispersion à une solution aqueuse de chitosane dans laquelle le chitosane a un degré d'acétylation compris entre 10 et 30 % et une masse moléculaire comprise entre 900 et 1 200 kilodaltons de manière à former un hydrogel chargé de l'ingrédient (des ingrédients) actif(s), et
d) récupérer l'hydrogel ainsi formé.

(a)

(b)

Figure 1

Figure 2

(a)

(b)

(c)

(d)

Figure 3

Figure 4

Figure 5

Figure 6

Figure 7

Figure 8

Figure 9

Figure 10

Figure 11

Figure 12